# EUROPEAN PATENT APPLICATION

(11) **EP 3 184 108 A1**
(43) Date of publication of application: **28.06.2017**
(21) Application number: 17150532.4
(22) Date of filing: 11.07.2007
(51) Int. Cl.: A61K 31/203, A61K 31/327, A61P 17/10

(54) **COMPOSITION COMPRISING A RETINOID AND BENZOYL PEROXIDE**

(30) Priority: 13.07.2006 FR 0652967; 21.07.2006 US 832092 P
(62) Divisional of application: 07787365.1
(71) Applicant: Galderma Research & Development, 06410 Biot (FR)
(72) Inventor: LOUIS, Fabienne, 06270 Villeneuve-Loubet (FR); WILLCOX, Nathalie, 06460 Saint Vallier De Thiey (FR); SEGURA-ORSONI, Sandrine, Bois Fleuri, 06410 Biot (FR)
(74) Representative: Casalonga

(57) **Abstract**

The present invention concerns a composition comprising, in a physiologically acceptable medium: at least one retinoid; benzoyl peroxide; and a gelling system comprising at least two categories of compounds, said categories of compounds being chosen from silicates, cellulose gelling agents and polysaccharide gums, on the condition that when at least two gelling agents are chosen from aluminium/magnesium silicate, xanthan gum and hydroxypropyl cellulose, the gelling system consists of these two gelling agents, or comprises at least one gelling agent different from these three compounds.

The present invention further concerns the use of said composition for manufacturing a pharmaceutical preparation intended to prevent and/or treat dermatological conditions such as acne vulgaris.

## Description

The invention relates to a composition comprising, in a physiologically acceptable medium, at least one retinoid, dispersed benzoyl peroxide and a gelling system comprising at least two particular categories of compounds.

The use of several classes of active principles is a therapeutic tool that is frequently employed, especially for treating dermatological disorders. Specifically, in the treatment of dermatitis it is known to use corticosteroids such as, for example hydrocortisone, miconazole or betamethasone valerate, antihistamines (e.g. mizolastine) and/or keratolytic agents such as salicylic acid. Various antifungal agents such as allylamine derivatives, triazoles, antibacterial or antimicrobial agents such as, for example, antibiotics, quinolones and imidazoles are also conventionally combined in the treatment of dermatological diseases. Peroxides, D vitamins and retinoids are also described for the topical treatment of various pathologies associated with the skin or mucous membranes, in particular acne.
The combination of several local treatments (antibiotics, retinoids, peroxides, zinc) is also used in dermatology to increase the effectiveness of the active principles and to decrease their toxicity (Cunliffe W.J., J. Dermatol. Treat., 2000, 11 (Suppl. 2), 513-514).
The multiple application of various dermatological products may be quite taxing and demanding for the patient.
The advantage is therefore understood in seeking to obtain a novel treatment that is effective on dermatological conditions, that has a stable composition offering good cosmetic quality, allowing a single application and a use that is pleasant for the patient.

However, the formulation of such a composition poses several problems.

Firstly, the effectiveness of benzoyl peroxide is linked to its decomposition when it is placed in contact with skin. Indeed, it is the oxidizing properties of the free radicals produced during this decomposition that result in the desired effect. Thus, in order to maintain an optimal effectiveness of the benzoyl peroxide, it is important to prevent its decomposition before use, that is to say during storage.
However, benzoyl peroxide is an unstable chemical compound which makes its formulation in finished products difficult.

The solubility and stability of benzoyl peroxide have been studied by Chellquist *et al.* in ethanol, propylene glycol and various mixtures of polyethylene glycol 400 (PEG 400) and water (Chellquist E.M. and Gorman W.G., Pharm. Res., 1992, Vol 9: 1341-1346). Benzoyl peroxide proved to be particularly soluble in PEG 400 and ethanol.

This document furthermore specifies that the stability of benzoyl peroxide is strongly influenced by the chemical composition of the formulation and by the storage temperature.
Benzoyl peroxide is extremely reactive and degrades in solution at low temperature due to the instability of its peroxide bond.
The authors thus report that benzoyl peroxide in solution degrades more or less quickly in all the solvents studied, depending on the type of solvent and on its concentration.

The degradation times of benzoyl peroxide in PEG 400 (0.5 mg/g), in ethanol and in propylene glycol are respectively 1.4, 29 and 53 days at 40°C. Such a degradation does not allow the preparation of a product intended for sale.

Furthermore it is known that benzoyl peroxide is more stable in water and propylene glycol when it is in suspension (i.e. in dispersed form), as it is not degraded after being kept for 90 days in these solvents.
Thus, to limit the problem of rapid instability of benzoyl peroxide in solution, it has proved advantageous to formulate the benzoyl peroxide in dispersed form.

Another difficulty to be overcome for preparing a composition comprising both benzoyl peroxide and a retinoid is that most retinoids are particularly sensitive to natural oxidation, to visible light and to ultraviolet light, and since benzoyl peroxide is a strong oxidizer the chemical compatibility of these compounds in one and the same formulation poses numerous stability problems from a physical and chemical viewpoint.

A study on the stability of two retinoids was carried out by combining two commercial products, one containing a retinoid (tretinoin or adapalene) and the second based on benzoyl peroxide (B. Martin et al., Br. J. Dermatol. (1998) 139, (suppl. 52), 8-11).
The presence of the formulation based on benzoyl peroxide causes a very rapid degradation of the oxidation-sensitive retinoids: it is calculated that 50% of the tretinoin is degraded in 2 hours and 95% in 24 hours. In the composition in which the retinoid is adapalene, no degradation of the adapalene was measured over 24 hours.

This study confirms that the benzoyl peroxide is degraded and degrades the oxidation-sensitive retinoids over time by gradually releasing benzoic acid into the initial products.
On the other hand, no indication was given regarding the physical stability of the two compositions brought together, nor on the therapeutic activity likely to be obtained at the end by combining the two active principles in the same composition.

Nothing was prompting these two active agents to be combined in order to obtain a stable gel-type composition, given that it was commonly known that the presence of benzoyl peroxide chemically and physically destabilized this type of composition.
However, it is clear that the too rapid degradation of benzoyl peroxide and the chemical degradation of the retinoids is undesirable in so far as it impairs the effectiveness of the composition containing them.

Furthermore, a finished product, in particular when it is a pharmaceutical or cosmetic composition, must keep, throughout its shelf life, precise physicochemical criteria that make it possible to guarantee its pharmaceutical or cosmetic quality respectively. Among these criteria, it is necessary that the rheological properties be retained. They define the behaviour and texture of the composition during application, but also the release properties of the active principle [1998 SFSTP Commission Report] and the homogeneity of the product when the active principles are present therein in the dispersed state.

In particular, the formulation of benzoyl peroxide and a retinoid in gel form is advantageous for topical treatments, such as those of acne, as it especially avoids letting a greasy feel remain on the skin.

Another difficulty to be overcome for preparing a composition especially comprising benzoyl peroxide, when it is in gel form, is that the gelling agents are destabilized by the benzoic acid released during the degradation of benzoyl peroxide.
Indeed, the most commonly used thickening agents for formulating these compositions with benzoyl peroxide are acrylic acid polymers (Carbomer).
However, the use of carbomers in aqueous gel-type compositions does not give good results in terms of chemical stability of benzoyl peroxide and in terms of rheological stability. As described by Bollinger (Bollinger, Journal of Pharmaceutical Science, 1977, vol. 5), a loss of 5 to 20% benzoyl peroxide has been observed at the end of 2 months at 40°C depending on the carbomer neutralizer used. Furthermore, the release of benzoic acid causes the depolymerization of the carbomers, giving a drop in viscosity which may result in phase separation.

This instability of benzoyl peroxide gels impairs their effectiveness and their cosmetic quality.

Therefore, the need remains to provide a physically stable gelled composition comprising benzoyl peroxide and a retinoid. However, nothing among the range of therapies proposed to a person skilled in the art would encourage him to combine, in the same composition, benzoyl peroxide and a retinoid and several different gelling agents.

However, the Applicant has surprisingly produced a composition satisfying this need, which comprises dispersed, free or encapsulated benzoyl peroxide, at least one retinoid and a gelling system comprising at least two categories of compounds, said composition having good physical stability, that is to say not having a drop in viscosity over time and at ambient temperature (between 20 and 30°C), and maintaining good chemical stability of the two active agents (benzoyl peroxide and retinoid). In particular, no degradation of the active agents is observed over time and/or at ambient temperature.

The invention therefore relates to a composition, especially a pharmaceutical composition, comprising, in a physiologically acceptable medium:
- at least one retinoid;
- benzoyl peroxide; and
- a gelling system comprising at least two categories of compounds,
said categories of compounds being chosen from silicates, cellulose gelling agents and polysaccharide gums,
on the condition that when at least two gelling agents are chosen from aluminium/magnesium silicate, xanthan gum and hydroxypropyl cellulose,
the gelling system consists of these two gelling agents, or comprises at least one gelling agent different from these three compounds.

The term "gelling system" is understood to mean a combination of gelling compounds that gives the composition a sufficient viscosity to keep the retinoid and the benzoyl peroxide in suspension, even under the influence, in particular, of a pH variation due to the release of benzoic acid by the benzoyl peroxide.

The term "physiologically acceptable medium" is understood to mean a medium that is compatible with the skin, the mucous membranes and/or the integuments.

The composition according to the invention is preferably in the form of an aqueous gel, which is particularly well suited to the target pathologies.

A gel is a system comprising at least one phase (in general one or two phases), which is thermodynamically stable, resulting from the coagulation of a colloidal solution into a three-dimensional network. More specifically, an aqueous gel corresponds to a composition containing, in an aqueous phase, a viscoelastic mass formed from colloidal suspensions.

More specifically, the gelling system comprises at least two categories of compounds, naturally different and distinct, said categories of compounds being chosen from silicates, cellulose gelling agents and polysaccharide gums.

The term "silicate" is understood in particular to mean clay derivatives, more specifically aluminium and magnesium silicates. The silicates are generally obtained from mineral clays having a crystalline lattice that are dissolved and purified in water to optimize the purity and effectiveness of the product. These compounds are especially solved by R.T. Vanderbilt under the name VEEGUM® (for example: VEEGUM® HV or VEEGUM® K).

The amount of silicate may vary over a wide range and depends in particular on the desired viscosity and on the other gelling agent and/or agents present in the composition. To give an order of magnitude, the silicate may be present in the composition according to the invention in concentrations between 0.1 and 10%, preferably ranging from 0.1 to 5% by weight relative to the total weight of the composition. Advantageously, the amount is between 0.5 and 2%, even more preferably between 0.8 and 1.8%, such as for example 1%, relative to the total weight of the composition.

The term "cellulose gelling agent" is understood to mean water-soluble polymers derived from cellulose. These polymers form water-soluble ethers (semisynthetic cellulose) originating from viscous solutions after dissolving in an aqueous solution. Among the cellulose polymers, mention may especially be made of methyl cellulose, ethyl cellulose, hydroxypropylmethyl cellulose, hydroxyethyl cellulose, hydroxylpropyl cellulose, carboxymethyl cellulose and hydroxymethyl cellulose. Preferably hydroxypropylmethyl cellulose or hydroxyethyl cellulose are used. These compounds are especially sold by Dow Chemical under the name METHOCEL® (for example: METHOCEL® E4M) or by Hercules under the name NATROSOL® (for example: NATROSOL® 250 HHX).

The amount of cellulose gelling agent may vary over a wide range and in particular depends on the desired viscosity and on the other gelling agent and/or agents present in the composition. To give an order of magnitude, the cellulose-based gelling agent may be present in the composition according to the invention in amounts between 0.1 and 10%, preferably ranging from 0.1 to 5% by weight relative to the total weight of composition. Advantageously, the amount is between 0.5 and 2%, such as for example 0.5%, 1%, 1.5% or 2% relative to the total weight of the composition.

Polysaccharide gums are complex mixtures of several high-molecular weight polysaccharides obtained by exudation from certain plants. Among the different types of polysaccharide gums, as non-limiting examples there is gum Arabic, gum tragacanth and xanthan gum, amongst another sold by CP Kelco under the name XANTURAL® (for example: XANTURAL® 180).

The amount of polysaccharide gum may vary over a wide range and depends in particular on the desired viscosity and on the other gelling agent and/or agents present in the composition. To give an order of magnitude, the polysaccharide gum may be present in the composition according to the invention in amounts between 0.01 and 5%, preferably ranging from 0.05 to 2% by weight relative to the total weight of the composition. Advantageously, the amount is between 0.1 and 0.8% such as, for example, 0.1%, 0.2% or 0.5% relative to the total weight of the composition.

The gelling system may comprise two categories of compounds or three categories of compounds, said categories of compounds being chosen from silicates, cellulose gelling agents and polysaccharide gums.

According to one particular aspect, the gelling system comprising two categories of compounds comprises at least one cellulose gelling agent (in particular hydroxypropylmethyl cellulose or hydroxyethyl cellulose) in combination with a silicate or a xanthan gum, taking into account the condition mentioned above.

According to another particular aspect, the gelling system comprising two categories of compounds comprises at least one silicate, in combination with a polysaccharide gum, taking into account the condition mentioned above.

According to another particular aspect, the gelling system comprising two categories of compounds comprises at least one polysaccharide gum, in combination with a cellulose gelling agent (in particular hydroxypropylmethyl cellulose or hydroxyethyl cellulose), taking into account the condition mentioned above.

The gelling system may also comprise at least one silicate, at least one cellulose gelling agent and at least one polysaccharide gum, taking into account the condition mentioned above.
Among the gelling systems having three categories of compounds, mention may especially be made of the combination of polysaccharide gum, silicate and a cellulose gelling agent, taking into account the condition mentioned above.

According to variants of the invention, the composition according to the invention may, in addition, comprise an additional gelling agent which may especially be chosen from mixtures of polyacrylamides such as the sodium acryloyldimethyltaurate copolymer/isohexadecane/polysorbate 80 mixture sold under the name SIMULGEL 600 by Seppic, the polyacrylamide/C13-14 isoparaffin/laureth-7 mixture such as, for example, that sold under the name SEPIGEL 305 by Seppic, the family of acrylic polymers coupled to hydrophobic chains such as the PEG-150/decyl/SMDI copolymer sold under the name ACULYN 44 (polycondensate comprising at least, as components, a polyethylene glycol having 150 or 180 moles of ethylene oxide, decyl alcohol and methylenebis(4-cyclohexylisocyanate) (SMDI), at 35% by weight in a mixture of propylene glycol (39%) and water (26%)), the family of modified starches such as the modified potato starch sold under the name STRUCTURE SOLANACE or else mixtures thereof.

The composition according to the invention contains at least one retinoid. The term "retinoid" is understood to mean any compound that binds to RAR and/or RXR receptors.

Preferably, the retinoid is a compound chosen from the family of benzonaphthalenic retinoids as described in Patent Application EP 0 199 636, and especially: 6-(3-methylphenyl)-2-naphthoic acid and its methyl ester;
6-(4-*tert*-butylphenyl)-2-naphthoic acid and its methyl ester;
6-(3-*tert*-butylphenyl)-2-naphthoic acid and its methyl ester;
6-(3,4-dimethoxyphenyl)-2-naphthoic acid and its methyl ester;
6-(p-(1-adamantylthio)phenyl)-2-naphthoic acid and its methyl ester;
6-(3-(1-adamantyl)-4-methoxyphenyl)-2-naphthoic acid (adapalene) and its methyl ester;
The methyl ester of 6-[3-(1-adamantyl)-4-*tert-*butyldimethylsilyloxyphenyl)-2-naphthoic acid;
The methyl ester of 6-[3-(1-adamantyl)-4-hydroxyphenyl)-2-naphthoic acid;
6-[3-(1-adamantyl)-4-hydroxyphenyl)-2-naphthoic acid; The methyl ester of 6-[3-(1-adamantyl)-4-decyloxyphenyl)-2-naphthoic acid;
6-[3-(1-adamantyl)-4-decyloxyphenyl)-2-naphthoic acid; The methyl ester of 6-[3-(1-adamantyl)-4-hexyloxyphenyl)-2-naphthoic acid;
6-[3-(1-adamantyl)-4-hexyloxyphenyl)-2-naphthoic acid; The methyl ester of 6-[3-(1-adamantyl)-4-methoxyphenyl)-4-acetoxy-1-methyl-2-naphthoic acid; 6-[3-(1-adamantyl)-4-methoxyphenyl)-4-hydroxy-1-methyl-2-naphthoic acid;
The methyl ester of 6-[3-(1-adamantyl)-4-methoxyphenyl)-4-hydroxy-1-methyl-2-naphthoic acid;
The methyl ester of 6-[3-(1-adamantyl)-4-methoxyphenyl)-1-methyl-2-naphthoic acid; 6-[3-(1-adamantyl)-4-methoxyphenyl)-1-methyl-2-naphthoic acid;
6-[3-(1-adamantyl)-4-methoxyphenyl)-2-naphthalene methanol;
The ethylamide of 6-[3-(1-adamantyl)-4-methoxyphenyl)-2-naphthoic acid;
The morpholide of 6-[3-(1-adamantyl)-4-methoxyphenyl)-2-naphthoic acid;
The methyl ester of 6-[3-*tert*-butyl-4-methoxyphenyl)-2-naphthoic acid;
6-[3-*tert*-butyl-4-methoxphenyl)-2-naphthoic acid;
The methyl ester of 6-[3-(1,1-dimethyldecyl)-4-methoxyphenyl)-2-naphthoic acid;
6-[3-(1,1-dimethyldecyl)-4-methoxyphenyl)-2-naphthoic acid.

In particular, adapalene and also its salts will be preferred.

The term "adapalene salts" is understood to mean the salt formed with a pharmaceutically acceptable base, especially mineral bases such as sodium hydroxide, potassium hydroxide and ammonia or organic bases such as lysine, arginine or N-methylglucamine.
The term "adapalene salts" is also understood to mean the salts formed with fatty amines such as dioctylamine and stearylamine.
Other retinoids may be chosen from those described in the following Patents or Patent Applications: US 4,666,941, US 4,581,380, EP 0 210 929, EP 0 232 199, EP 0 260 162, EP 0 292 348, EP 0 325 540, EP 0 359 621, EP 0 409 728, EP 0 409 740, EP 0 552 282, EP 0 584 191, EP 0 514 264, EP 0 514 269, EP 0 661 260, EP 0 661 258, EP 0 658 553, EP 0 679 628, EP 0 679 631, EP 0 679 630, EP 0 708 100, EP 0 709 382, EP 0 722 928, EP 0 728 739, EP 0 732 328, EP 0 740 937, EP 0 776 885, EP 0 776 881, EP 0 823 903, EP 0 832 057, EP 0 832 081, EP 0 816 352, EP 0 826 657, EP 0 874 626, EP 0 934 295, EP 0 915 823, EP 0 882 033, EP 0 850 909, EP 0 879 814, EP 0 952 974, EP 0 905 118, EP 0 947 496, WO98/56783, WO99/10322, WO99/50239, WO 99/65872.

Of course, the amount of the two active agents, benzoyl peroxide and retinoid, in the composition according to the invention, will depend on the combination chosen and therefore particularly on the retinoid in question and on the quality of the desired treatment.

The preferred retinoid concentrations are between 0.0001 and 20% by weight relative to the total weight of the composition.

Benzoyl peroxide could also be used in the free form or else in an encapsulated form in a form adsorbed on, or absorbed in, any porous support.
It could for example be benzoyl peroxide encapsulated in a polymer system made of porous microspheres, such as for example microsponges sold under the name MICROSPONGES P009A Benzoyl Peroxide by Cardinal Healthcare.

To give an order of magnitude, the composition according to the invention advantageously comprises between 0.0001 and 20% by weight of benzoyl peroxide and between 0.0001 and 20% by weight of retinoid relative to the total weight of the composition, and preferably, respectively, between 0.025 and 10% by weight of benzoyl peroxide and between 0.001 and 10% by weight of retinoid relative to the total weight of the composition.

For example, in compositions for treating acne, benzoyl peroxide is preferably used at concentrations ranging from 2 to 10% by weight and more particularly from 2.5 to 5% by weight relative to the total weight of the composition. Retinoid is itself used in this type of composition at concentrations generally ranging from 0.05 to 1% by weight relative to the total weight of the composition.

Advantageously, the particle size of the retinoid and of the benzoyl peroxide is such that at least 80% by number of particles, and preferably at least 90% by number of particles, have a diameter of less than 25 µm and at least 99% by number of particles have a diameter of less than 100 µm.

According to the invention, advantageously, the gel containing the benzoyl peroxide and a retinoid comprises at least water and may also comprise a propenetrating agent and/or a wetting liquid surfactant.

The compositions of the invention may contain one or more propenetrating agents in preferable concentrations ranging from 0.01 to 20% and more preferably ranging from 2 to 6% by weight relative to the total weight of the composition. Preferably 2, 4 and 5%.

The propenetrating agents should generally not dissolve the active agents at the percentage used, should not cause exothermic reactions that damage the benzoyl peroxide, should help in the satisfactory dispersion of the active agents and should have antifoaming properties. Among the propenetrating agents preferably used, without this list being limiting, are compounds such as propylene glycol, dipropylene glycol, propylene glycol dipelargonate, lauroglycol and ethoxydiglycol. The particularly preferred propenetrating agent is propylene glycol.

Advantageously, the compositions according to the invention may also contain one or more wetting liquid surfactants in preferable concentrations ranging from 0.01 to 10% and more preferably ranging from 0.1 to 2%. The wetting ability is the tendency of a liquid to spread out over a surface.
Preferably, they are surfactants having a HLB (Hydrophillic Lipophilic Balance) of 7 to 12, or else non-ionic surfactants of polyoxyethylenated and/or polyoxypropylenated copolymer type. They should be liquid so as to easily be incorporated into the composition without it being necessary to heat them, the objective being, amongst other things, to obtain a low-temperature manufacturing method, which in no way limits the present invention.
Among the wetting agents preferably used, without this list being limiting, are compounds of the family of poloxamers and more particularly Poloxamer 124 and/or Poloxamer 182.

The particularly preferred wetting liquid surfactant is Poloxamer 124.

The composition may comprise, in addition, any additive commonly used in the cosmetic or pharmaceutical field, such as sequestrants, antioxidants, sunscreens, preservatives, fillers, electrolytes, humectants, dyes, common mineral or organic bases or acids, fragrances, essential oils, cosmetic active agents, moisturizers, vitamins, essential fatty acids, sphingolipids, self-tanning compounds such as DHA and skin soothing and protective agents such as allantoin.

Of course, a person skilled in the art will be sure to choose this or these optional additional compounds, and/or their amount, so that the advantageous properties of the composition according to the invention are not, or are not substantially, adversely affected.

These additives may be present in the composition in an amount of 0.001 to 20% by weight relative to the total weight of the composition.

Mention may be made, as examples of sequestering agents, of ethylenediamine tetracetic acid (EDTA), and also derivatives or salts thereof, dihydroyethylglycine, citric acid, tartric acid, or mixtures thereof.
Mention may be made, as examples of preservatives, of benzalkonium chloride, phenoxyethanol, benzyl alcohol, diazolidinylurea, parabens, or mixtures thereof. Mention may be made, as examples of humectants, of glycerol and sorbitol.

In particular, the invention also relates to a pharmaceutical or cosmetic composition for topical application to the skin, integuments or mucous membranes, in the form of an aqueous gel, characterized in that it comprises, in a physiologically acceptable medium that is compatible with topical application to the skin, integuments or mucous membranes, an active phase comprising (expressed as percentage by weight):
- 20 to 90% of water;
- 0 to 10%, preferably 0 to 2%, especially 0 to 0.5% of wetting liquid surfactant;
- 0 to 20%, preferably 0 to 10%, especially 2 to 5% of propenetrating agent;
- 0.0001 to 20%, preferably 0.025 to 10% of benzoyl peroxide;
- 0.0001 to 20%, preferably 0.001 to 10% of retinoid; and
- 0.11 to 25% of a gelling system as described above.

The aqueous phase of the composition according to the invention may comprise water, a floral water such as cornflower water, or a spring or natural mineral water, for example chosen from water from Vittel, waters from the Vichy basin, water from Uriage, water from la Roche Posay, water from la Bourboule, water from Enghien-les-Bains, water from Saint Gervais-les-Bains, water from Néris-les-Bains, water from Allevard-les-Bains, water from Digne, water from Maizières, water from Neyrac-les-Bains, water from Lons-le-Saunier, water from Bonnes, water from Rochefort, water from Saint Christau, water from Furnades and water from Tercis-les-Bains, water from Avène or water from Aix les Bains.

Said aqueous phase may be present in an amount between 10 and 90% by weight relative to the total weight of the composition, preferably between 20 and 85% by weight.

A preferred composition according to the invention comprises, in water:
- 2.5% of benzoyl peroxide;
- 0.1% of adapalene;
- 0.1% of disodium EDTA;
- 2%-4% of glycerol;
- 2%-6%; (preferably 2%, 4% or 5%) of propylene glycol
- 0%-0.05% of sodium docusate;
- 2%-4% of a gelling system as described above; and
- 0.2%-0.5% of Poloxamer 124.

A particularly preferred composition according to the invention comprises, in water:

| | |
|---|---|
| Adapalene | 0.1% |
| Benzoyl peroxide | qs 2.5% BPO |
| Propylene glycol | 4% |
| Poloxamer 124 | 0.1% |
| Aluminium/magnesium silicate | 2% |
| Hydroxyethyl cellulose | 1.5% |
| Xanthan gum | 0.5% |
| Glycerol | 2% |
| Sodium docusate | 0.05% |

The methods for preparing the composition according to the invention are conventional methods that can optionally be adapted by a person skilled in the art.

Another subject of the present invention is the composition as described previously as medication.

The invention also relates to the use of the composition as described previously in cosmetics or in dermatology.

On account of the keratolytic, bactericidal and antiinflammatory activity of benzoyl peroxide and the pronounced activity of retinoids in the fields of cell differentiation and proliferation, the compositions of the invention are particularly suitable in the following therapeutic fields:
1) for treating dermatological conditions associated with a keratinization disorder relating differentiation and proliferation, especially for treating acne vulgaris and comedonal, polymorphous and rosacea acnes, nodulocystic acne and acne conglobara, senile acne, secondary acne such as solar, drug or occupational acne and hidradenitis suppurativa;
2) for treating other types of keratinization disorders, especially ichthyosis, ichthyosiform states, Darrier's disease, palmoplantar keratoderma, leukoplakia or leucoplakiform states, and cutaneous or mucous (buccal) lichen;
3) for treating other dermatological conditions associated with a keratinization disorder having an inflammatory and/or immuno-allergic component and especially all forms of psoriasis whether they are cutaneous, mucous or ungula, and even psoriatic rheumatism, or else cutaneous atopy, such as eczema or respiratory atopy or else gingival hypertrophy; the compounds may also be used in certain inflammatory conditions that do not exhibit keratinization disorders, such as folliculitis;
4) for treating all dermal or epidermal proliferations, whether benign or malignant, whether or not they are of viral origin, such as verrucas, plane warts, molluscum contagiosum and epidermodysplasia verruciformis, oral or floride papillomatoses and proliferations which may be induced by ultraviolet light especially in the case of actinic keratoses;
5) for repairing or combating skin aging, whether light-induced or chronological, or for reducing pigmentation, or any pathologies associated with chronological and actinic aging;
6) for treating wound-healing disorders or skin ulcers in a preventative or curative manner, for preventing or repairing striae atrophicae, or else for promoting wound healing;
7) for combating sebaceous gland disorders such as hyperseborrhea of acne or simple seborrhea;
8) in treating any skin condition of fungal origin, such as tinea pedis and tinea versicolor;
9) in the treatment of dermatological conditions having an immunological component;
10) in the treatment of skin disorders due to exposure to UV radiation; and
11) in the treatment of dermatological conditions associated with an inflammation or infection of the tissues surrounding the hair follicle, especially due to a microbial colonization or infection, especially impetigo, seborrheic dermatitis, folliculitis, sycosis barbae or those involving any other bacterial or fungal agent.

The compositions according to the invention are particularly suitable to the treatment, in a preventative and/or curative manner, of acne vulgaris.

One subject of the invention also relates to the manufacture of a pharmaceutical preparation intended for preventing and/or treating dermatological conditions linked to cell differentiation and/or proliferation disorders and/or keratinization disorders, more particularly to the manufacture of a pharmaceutical preparation intended to prevent and/or treat acne vulgaris.

The compositions according to the invention also find an application in body and hair hygiene.

The compositions according to the invention particularly find an application in the cosmetics field, in particular for treating acne-prone skin, for hair regrowth, for preventing hair loss, for combating the greasy appearance of the skin or hair, in protecting against the damaging effects of the sun or in treating physiologically greasy skin, or for preventing and/or combating light-induced or chronological aging.

The examples below make it possible to illustrate the invention without however limiting the scope thereof.

### Examples

### Example 1: duos of gelling agents

### a) Silicate/gum tragacanth

| | |
|---|---|
| Water | qs 100% |
| Adapalene | 0.1% |
| Benzoyl peroxide | qs 2.5% BPO |
| Propylene glycol | 2% |
| Poloxamer 124 | 0.2% |
| Disodium EDTA | 0.1% |
| Silicate (Veegum K) | 2% |
| Gum tragacanth | 0.5% |
| Glycerol | 2% |
| Sodium docusate | 0.05% |

### b) Xanthan gum/cellulose (hydroxyethyl cellulose)

| | |
|---|---|
| Water | qs 100% |
| Adapalene | 0.1% |
| Benzoyl peroxide | qs 2.5% BPO |
| Propylene glycol | 4% |
| Poloxamer 124 | 0.2% |
| Disodium EDTA | 0.1% |
| Hydroxyethyl cellulose (Natrosol HHX) | 2% |
| Xanthan gum (Xantural 18) | 0.2% |
| Glycerol | 4% |

### c) Cellulose/silicate

| | |
|---|---|
| Water | qs 100% |
| Adapalene | 0.1% |
| Benzoyl peroxide | qs 2.5% BPO |
| Dipropylene glycol | 4% |
| Poloxamer 124 | 0.2% |
| Disodium EDTA | 0.1% |
| Hydroxyethyl cellulose (Natrosol HHX) | 2% |
| Silicate (Veegum HV) | 1% |
| Glycerol | 4% |
| Sodium docusate | 0.05% |

### Example 2: trios of gelling agents:

### a) Silicate/cellulose/xanthan gum

| | |
|---|---|
| Water | qs 100% |
| Adapalene | 0.1% |
| Benzoyl peroxide | qs 2.5% BPO |
| Propylene glycol | 4% |
| Poloxamer 124 | 0.2% |
| Disodium EDTA | 0.1% |
| Veegum K | 2% |
| Hydroxyethyl cellulose (Natrosol HHX) | 1.5% |
| Xanthan gum (Xantural 180) | 0.5% |
| Glycerol microsponges | 4% |
| Glycerol | 2% |
| Sodium docusate | 0.05% |

### Specifications at T0

*Macroscopic appearance* : White, smooth and shiny product
*Microscopic appearance* : Good dispersion of both active agents.

### PHYSICAL STABILITY :

| Time→ | T 1 month | T 2 months | T 3 months |
|---|---|---|---|
| Stability conditions↓ | | | |
| TA | Conforms to specifications | Conforms to specifications | Conforms to specifications |
| 40°C | Conforms to specifications | Conforms to specifications Slightly yellowing of the product | Conforms to specifications Slightly yellowing of the product |

### CHEMICAL STABILITY:

ADAPALENE : Assay of the active by internal calibration in HPLC.

At the starting time (TO) the composition is considered to contain 100% of adapalene. Concentration of adapalene measured in % relative to T0:

| Time→ | T 0 | T 1 month | T 2 months | T 3 months |
|---|---|---|---|---|
| Stability conditions↓ | | | | |
| AT | NR | NR | NR | 97,7% |
| 40°C | | NR | NR | NR |

BENZOYL PEROXIDE : Assay of the active by internal calibration in HPLC.

At the starting time (TO) the composition is considered to contain 100% of benzoyl peroxide. Concentration of benzoyl peroxide measured in % relative to T0:

| Time→ | T 0 | T 1 month | T 2 months | T 3 months |
|---|---|---|---|---|
| Stability conditions↓ | | | | |
| AT | 99.2 | NR | NR | 94.8 |
| 40°C | | 96.3 | 81.6% | 88.7 |

### b) Silicate/cellulose/xanthan gum

| | |
|---|---|
| Water | qs 100% |
| Adapalene | 0.1% |
| Benzoyl peroxide | qs 2.5% BPO |
| Propylene glycol | 5% |
| Poloxamer 124 | 0.5% |
| Disodium EDTA | 0.1% |
| Veegum K | 2% |
| Hydroxyethyl cellulose (Methocel E4M) | 0.5% |
| Xanthan gum (Xantural 180) | 0.1% |
| Glycerol | 4% |
| Sodium docusate | 0.05% |

### c) Silicate/cellulose/gum arabic

| | |
|---|---|
| Water | qs 100% |
| Adapalene | 0.1% |
| Benzoyl peroxide | qs 2.5% BPO |
| Propylene glycol | 5% |
| Poloxamer 124 | 0.5% |
| Disodium EDTA | 0.1% |
| Veegum K | 2% |
| Carboxymethyl cellulose | 0.5% |
| Gum arabic | 0.1% |
| Glycerol | 4% |
| Sodium docusate | 0.05% |

## Claims

1. Composition comprising, in a physiologically acceptable medium:
- at least one retinoid;
- benzoyl peroxide; and
- a gelling system comprising at least two categories of compounds,
said categories of compounds being chosen from silicates, cellulose gelling agents and polysaccharide gums,
on the condition that when at least two gelling agents are chosen from aluminium/magnesium silicate, xanthan gum and hydroxypropyl cellulose,
the gelling system consists of these two gelling agents, or comprises at least one gelling agent different from these three compounds.

2. Composition according to Claim 1, **characterized in that** the silicates are magnesium and aluminium silicates.

3. Composition according to either of Claims 1 and 2, **characterized in that** the amount of silicate is between 0.1 and 10% by weight relative to the total weight of the composition.

4. Composition according to one of Claims 1 to 3, **characterized in that** the cellulose gelling agents are chosen from methyl cellulose, ethyl cellulose, hydroxypropyl methyl cellulose, hydroxyethyl cellulose, carboxymethyl cellulose, hydroxymethyl cellulose and hydroxypropyl cellulose.

5. Composition according to any one of the preceding claims, **characterized in that** the amount of cellulose gelling agent is between 0.1 and 10% by weight relative to the total weight of the composition.

6. Composition according to any one of the preceding claims, **characterized in that** the amount of polysaccharide gum is between 0.01 and 5% by weight relative to the total weight of the composition.

7. Composition according to any one of the preceding claims, **characterized in that** the gelling system comprises at least one cellulose gelling agent in combination with a silicate.

8. Composition according to any one of Claims 1 to 6, **characterized in that** the gelling system comprises at least one silicate in combination with a polysaccharide gum.

9. Composition according to any one of Claims 1 to 6, **characterized in that** the gelling system comprises at least one polysaccharide gum in combination with a cellulose gelling agent.

10. Composition according to any one of the preceding claims, **characterized in that** the gelling system comprises at least one silicate, at least one cellulose gelling agent and at least one polysaccharide gum.

11. Composition according to any one of the preceding claims, **characterized in that** it contains between 0.0001 and 20% of retinoid.

12. Composition according to any one of the preceding claims, **characterized in that** the retinoid is adapalene.

13. Composition according to one of the preceding claims, **characterized in that** it contains between 0.0001 and 20% of benzoyl peroxide.

14. Composition according to one of the preceding claims, **characterized in that** the benzoyl peroxide is encapsulated or free.

15. Composition according to any one of the preceding claims, **characterized in that** it comprises a propenetrating agent.

16. Composition according to Claim 15, **characterized in that** the propenetrating agent is propylene glycol.

17. Composition according to any one of the preceding claims, **characterized in that** it comprises a wetting liquid surfactant.

18. Composition according to Claim 17, **characterized in that** the wetting liquid surfactant is a poloxamer.

19. Composition according to any one of the preceding claims, **characterized in that** it comprises, in water:
- 2.5% of benzoyl peroxide;
- 0.1% of adapalene;
- 0.1% of disodium EDTA;
- 2%-4% of glycerol;
- 2%-6% of propylene glycol;
- 0%-0.05% of sodium docusate;
- 2%-4% of a gelling system; and
- 0.2%-0.5% of Poloxamer 124.

20. Composition according to any one of the preceding claims, **characterized in that** it comprises, in water:
| | |
|---|---|
| Adapalene | 0.1% |
| Benzoyl peroxide | qs 2.5% BPO |
| Propylene glycol | 4% |
| Poloxamer 124 | 0.1% |
| Aluminium/magnesium silicate | 2% |
| Hydroxyethyl cellulose | 1.5% |
| Xanthan gum | 0.5% |
| Glycerol | 2% |
| Sodium docusate | 0.05% |

21. Composition according to any one of the preceding claims as medication.

22. Use of a composition according to any one of claims 1 to 20 for manufacturing a pharmaceutical preparation intended to prevent and/or treat the dermatological conditions linked to cell differentiation and/or proliferation disorders and/or keratinization disorders.

23. Use of a composition according to Claim 22 for preparing a pharmaceutical preparation intended to prevent and/or treat acne vulgaris.

24. Cosmetic use of a composition according to any one of Claims 1 to 20 for treating acne-prone skin, for hair regrowth, for preventing hair loss, for combating the greasy appearance of the skin or hair, in protecting against the damaging effects of the sun or in treating physiologically greasy skin, or for preventing and/or combating light-induced or chronological aging.
